## Europäisches Patentamt
## European Patent Office
## Office européen des brevets

(11) Publication number: **0 010 437**
**B1**

(12) **EUROPEAN PATENT SPECIFICATION**

(45) Date of publication of patent specification: **08.08.84**

(51) Int. Cl.³: **A 61 K 9/08,** A 61 K 7/48, A 61 K 31/71

(21) Application number: **79302271.6**

(22) Date of filing: **19.10.79**

(54) **Stable erythromycin solution and process therefor.**

(30) Priority: **20.10.78 US 953159**

(43) Date of publication of application:
**30.04.80 Bulletin 80/9**

(45) Publication of the grant of the patent:
**08.08.84 Bulletin 84/32**

(84) Designated Contracting States:
**DE IT NL SE**

(56) References cited:
**FR - A - 2 378 523**
**US - A - 4 000 263**

(73) Proprietor: **ELI LILLY AND COMPANY**
**307, East McCarty Street**
**Indianapolis Indiana 46285 (US)**

(72) Inventor: **Takruri, Harun**
**24222 Tahoe Court**
**Laguna Niguel California 92677 (US)**
Inventor: **Chavers, Jane Ellen**
**8515 Canterbury Square West**
**Indianapolis Indiana 46260 (US)**

(74) Representative: **Crowther, Terence Roger**
**European Patent Attorney et al,**
**Erl Wood Manor**
**Windlesham Surrey GU20 6PH (GB)**

The file contains technical information
submitted after the application was filed and not
included in this specification

Courier Press, Leamington Spa, England.

## Description

Erythromycin has a limited stability in aqueous solution according to *Chemical Abstracts, 60,* 14337 (1964). US—A— 4,000,263 discloses a stable erythromycin solution containing propylene glycol, ethyl alcohol and an ethoxylated ether of lauryl alcohol (Brij-30). The amount of propylene glycol varies from 35 to 45 percent. According to US—A— 4,000,263 the erythromycin solutions disclosed therein lose about 5% of their stability in 10 weeks at 45°C. or in 18 weeks at 37°C.

Mills, writing in *Cutis, 15,* 93 (1975), described the use of a 2 percent solution of erythromycin in a 1:1 ethanol-water solvent for treatment of acne. J. E. Fulton Jr., *Dermat, 110,* 83 (1974), used a 1 percent solution of erythromycin in a solvent containing two parts of ethanol, 2 parts ethylene glycol monomethyl ether and 1 part of propylene glycol. The author reported this preparation is quite effective for the treatment of acne.

FR—A—2,378,523 discloses erythromycin preparations for the treatment of acne and as excipients for an alcoholic lotion form of such preparation are given ethanol and propylene glycol. The preparation can additionally contain vitamin A acid and/or benzoyl peroxide.

Antibiotics, including members of the lincomycin family, tetracycline and epi-tetracycline, have been used to treat acne; see U.S. Patent Nos. 3,969,516 and 3,952,099. The latter patent is more concerned with increased penetration of the antibiotic through the skin by the addition of decylmethylsulfoxide to an alcoholic solution of the antibiotic.

The invention provides a pharmaceutical formulation comprising per ml. from 1 to 100 mg. of the active ingredient erythromycin and the excipients ethanol sufficient ctric acid to give a pH in the range of 8—10 after dilution with water, and propylene glycol not exceeding 0.3 ml. but sufficient to bring the volume to 1 ml.

The invention also provides a process for the preparation of a pharmaceutical formulation having as active ingredient erythromycin comprising dissolving 1 to 100 mg. per ml. final volume erythromycin in ethanol, adding sufficient citric acid to give a pH in the range of 8—10 after dilution with water and adding propylene glycol in an amount of up to 30% of the final volume of the preparation. It is preferred for the ethanol to be present in an amount corresponding to 70% of the final volume of the preparation.

The preferred concentration of erythromycin in the solution is 20 mg. per ml. In the preparation of the solution sufficient citric acid is added to give a final pH in the range 8—10 upon dissolving the preparation in sufficient water to enable a pH reading to be taken. The pH is suitably determined on a pH meter with a glass electrode. It has been found that a final concentration of 0.04% of citric acid (0.4 mg/ml) gives a solution which upon dilution in water yields of pH of about 9 with a glass electrode.

A double-blind study was carried out in humans suffering from acne in which the vehicle itself lacking the erythromycin was used as a control. Those patients receiving a formulation made in accordance with this invention showed a 56% decrease in the number of papules over the test period whereas in the control group which received the formulation vehicle alone there was only a 31% decrease in papules. The number of patients used were sufficient to render this difference statistically significant.

The solutions of this invention are quite stable. For example, a solution containing 22 mg of erythromycin (10% excess), 0.7 ml of ethanol USP, sufficient citric acid to give a final concentration of 0.04% and propylene glycol q.s. to 1 ml. was shown to assay 83.2% of initial (92.9% of label claim) antibiotic present at 25°C after thirty months storage. Other solutions confirm this trend in stability.

In preparing the erythromycin solution of this invention, it is customary to dissolve the erythromycin base and citric acid in ethanol and then add sufficient propylene glycol to make up the final solution to 1 ml or a multiple thereof. The ingredients are then thoroughly mixed.

Whilst the propylene glycol can be present in an amount of up to 30% of the total volume of formulation (0.3 ml per ml), it is preferably present in an amount which itself is at least sufficient to dissolve the erythromycin contained in the formulation so that the erythromycin is kept in solution and available for absorption into the skin upon any evaporation of the ethanol. Amounts of above 30% propylene glycol tend to cause skin irritation. The ethanol is likewise preferably present in an amount at least sufficient to dissolve the erythromycin contained in the formulation.

## Claims

1. A liquid pharmaceutical formulation comprising:

(A) erythromycin in an amount of from 1 to 100 mg per ml of formulation,
(B) ethanol in an amount at least sufficient in itself to dissolve the amount of erythromycin contained in the formulation,
(C) propylene glycol in an amount at least sufficient in itself to dissolve the amount of erythromycin contained in the formulation, subject to the proviso that the propylene glycol is present in an amount not exceeding 30% by volume of the formulation, and
(D) citric acid in an amount to give a pH value in the range of 8 to 10 upon sufficient dilution of the formulation with water to enable a pH reading with a glass electrode to be obtained.

2. A liquid pharmaceutical formulation according to claim 1, wherein the citric acid is present in an amount of about 0.4 mg per ml of formulation.

3. A liquid pharmaceutical formulation according to claim 1 or 2, wherein the erythromycin is present in an amount of 20 mg per ml of formulation.

4. A liquid pharmaceutical formulation according to any preceding claim when the ethanol is present in an amount of 0.7 ml per ml of formulation.

5. A process for the preparation of a pharmaceutical formulation according to claim 1 which comprises dissolving 1 to 100 mg per ml of final formulation of erythromycin in ethanol, adding sufficient citric acid to give a pH value to the final formulation in the range of 8 to 10 upon sufficient dilution of the final formulation with water to enable a pH reading with a glass electrode to be obtained and adding propylene glycol in an amount at least sufficient in itself to dissolve the amount of erythromycin contained in the formulation, provided that the propylene glycol is added in an amount not exceeding 30% by volume of the final formulation.

6. A process according to claim 5, wherein the citric acid is added in an amount corresponding to 0.4 mg per ml of final formulation.

7. A process according to claim 5 or 6, wherein the ethanol is employed in an amount corresponding to 70% of the final volume of the formulation.

8. A process according to any one of claims 5 to 7, wherein the erythromycin is employed in an amount corresponding to 20 mg per ml of final formulation.

9. A liquid pharmaceutical formulation according to any one of claims 1 to 4 for use in the treatment of acne.

**Patentansprüche**

1. Flüssige pharmazeutische Formulierung, dadurch gekennzeichnet, daß sie besteht aus

(A) Erythromycin in einer Menge von 1 bis 100 mg pro ml Formulierung,
(B) Ethanol in einer Menge, die wenigstens zum Auflösen der Menge an in der Formulierung enthaltenem Erythromycin ausreicht,
(C) Propylenglykol in einer Menge, die wenigstens zum Auflösen der in der Formulierung enthaltenen Menge an Erythromycin ausreicht, mit der Maßgabe, daß das Propylenglykol in einer Menge vorhanden ist, die nicht mehr als 30 Vol.-% der Formulierung ausmacht, und
(D) Citronensäure in einer Menge, die einen pH-Wert im Bereich von 8 bis 10 ergibt, nachdem die Formulierung so stark mit Wasser verdünnt worden ist, daß sich ihr pH-Wert mit einer Glaselektrode ablesen läßt.

2. Flüssige pharmazeutische Formulierung nach Anspruch 1, dadurch gekennzeichnet, daß die Citronensäure in einer Menge von etwa 0,4 mg pro ml Formulierung vorhanden ist.

3. Flüssige pharmazeutische Formulierung nach Anspruch 1 oder 2, dadurch gekennzeichnet, daß das Erythromycin in einer Menge von 20 mg pro ml Formulierung vorhanden ist.

4. Flüssige pharmazeutische Formulierung nach einem der vorhergehenden Ansprüche, dadurch gekennzeichnet, daß sie das Ethanol in einer Menge von 0,7 ml pro ml Formulierung enthält.

5. Verfahren zur Herstellung einer pharmazeutischen Formulierung nach Anspruch 1, dadurch gekennzeichnet, daß man 1 bis 100 mg Erythromycin pro ml fertiger Formulierung in Ethanol löst, die Ethanollösung mit einer solchen Menge Citronensäure versetzt, daß der pH-Wert der fertigen Formulierung im Bereich von 8 bis 10 liegt, nachdem die Formulierung so stark mit Wasser verdünnt worden ist, daß sich ihr pH-Wert mit einer Glaselektrode ablesen läßt, und die erhaltene citronensaure Lösung mit einer Menge an Propylenglykol versetzt, die wenigstens zum Auflösen der Menge des in der Formulierung enthaltenen Erythromycins ausreicht, mit der Maßgabe, daß man das Propylenglykol in einer Menge zusetzt, die nicht mehr als 30 Vol.-% der fertigen Formulierung ausmacht.

6. Verfahren nach Anspruch 5, dadurch gekennzeichnet, daß man die Citronensäure in einer Menge zusetzt, die 0,4 mg pro ml der fertigen Formulierung entspricht.

7. Verfahren nach Anspruch 5 oder 6, dadurch gekennzeichnet, daß man das Ethanol in einer Menge einsetzt, die 70% des fertigen Volumens der Formulierung entspricht.

8. Verfahren nach einem der Ansprüche 5 bis 7, dadurch gekennzeichnet, daß man das Erythromycin in einer Menge verwendet, die 20 mg pro ml der fertigen Formulierung entspricht.

9. Verwendung der flüssigen pharmazeutischen Formulierung nach einem der Ansprüche 1 bis 4 zur Behandlung von Akne.

**Revendications**

1. Formulation pharmaceutique liquide comprenant:

(A) de l'érythromycine en une quantité de 1 à 100 mg par ml de formulation,
(B) de l'éthanol en une quantité au moins suffisante en elle-même pour dissoudre la quantité d'érythromycine contenue dans la formulation,
(C) du propylène-glycol en une quantité au moins suffisante en elle-même pour dissoudre la quantité d'érythromycine contenue dans la formulation, avec cette réserve que le propylène-glycol est présent en une quantité ne dépassant pas 30% en volume de la formulation, et

(D) de l'acide citrique en une quantité permettant d'atteindre un pH se situant dans l'intervalle allant de 8 à 10 lors d'une dilution suffisante de la formulation avec de l'eau afin de pouvoir effectuer une lecture du pH avec une électrode en verre.

2. Formulation pharmaceutique liquide suivant la revendication 1, caractérisée en ce que l'acide citrique est présent en une quantité d'environ 0,4 mg par ml de formulation.

3. Formulation pharmaceutique liquide suivant la revendication 1 ou 2, caractérisée en ce que l'érythromycine est présente en une quantité de 20 mg par ml de formulation.

4. Formulation pharmaceutique liquide suivant l'une quelconque des revendications précédentes, caractérisée en ce que l'éthanol est présent en une quantité de 0,7 ml par ml de formulation.

5. Procédé de préparation d'une formulation pharmaceutique suivant la revendication 1, caractérisé en ce qu'il comprend les étapes qui consistent à dissoudre 1 à 100 mg d'érythromycine par ml de formulation dans de l'éthanol, ajouter de l'acide citrique en une quantité suffisante pour conférer, à la formulation finale, un pH se situant dans l'intervalle allant de 8 à 10 lors d'une dilution suffisante de la formulation finale avec de l'eau afin de pouvoir effectuer une lecture du pH avec une électrode en verre et ajouter du propylèneglycol en une quantité au moins suffisante en elle-même pour dissoudre la quantité d'érythromycine contenue dans la formulation, avec cette réserve que le propylène-glycol est ajouté en une quantité ne dépassant pas 30% en volume de la formulation finale.

6. Procédé suivant la revendication 5, caractérisé en ce que l'acide citrique est ajouté en une quantité correspondant à 0,4 mg/ml de la formulation finale.

7. Procédé suivant la revendication 5 ou 6, caractérisé en ce que l'éthanol est utilisé en une quantité correspondant à 70% du volume final de la formulation.

8. Procédé suivant l'une quelconque des revendications 5 à 7, caractérisé en ce que l'érythromycine est utilisée en une quantité correspondant à 20 mg/ml de la formulation finale.

9. Formulation pharmaceutique liquide suivant l'une quelconque des revendications 1 à 4 en vue de l'utiliser pour le traitement de l'acné.